# FASCICULE DE BREVET EUROPEEN

(11) **EP 2 958 529 B1**
(45) Date de publication et mention de la délivrance du brevet: **26.07.2017**
(21) Numéro de dépôt: 14711811.1
(22) Date de dépôt: 19.02.2014
(51) Int. Cl.: A61F 5/00, A63B 71/12, A47C 7/02, A41D 1/08

(54) **DISPOSITIF DE COQUE PROTECTRICE ERGONOMIQUE DE TYPE ORTHESE PERINEALE**
ERGONOMISCHE SCHUTZHÜLSENVORRICHTUNG FÜR PERINEALORTHESEN
ERGONOMIC PROTECTIVE SHELL DEVICE OF THE PERINEAL ORTHOSIS TYPE

(30) Priorité: 20.02.2013 FR 1351441; 05.06.2013 FR 1355163
(43) Date de publication de la demande: 30.12.2015
(73) Titulaire: Claripharm, 22400 St Alban (FR)
(72) Inventeur: GIQUEL, Loïc, F-34090 Montpellier (FR); LAGADEUC, Clarisse, F-22370 Pleneuf Val Andre (FR)
(74) Mandataire: Rhein, Alain
(86) Numéro de dépôt international: PCT/FR2014/050344
(87) Numéro de publication internationale: WO 2014/128405

(56) Documents cités:
- EP-A1- 0 448 336
- EP-A1- 2 191 746
- CN-Y- 201 127 648
- US-A- 4 186 739
- US-A- 5 483 705
- US-A- 5 978 970
- US-A1- 2002 124 318
- US-A1- 2006 229 546
- US-A1- 2008 229 486

## Description

La présente invention entre dans le domaine médical et paramédical pour la prévention de la douleur et l'amélioration du confort d'un patient, préférentiellement d'une patiente.

L'invention trouvera une application particulière dans le cadre d'un patient, préférentiellement d'une patiente, ayant subi une intervention médicale, notamment chirurgicale, souffrant de douleurs à l'entrejambe suite à une telle intervention ou en raison d'une pathologie.

En particulier, mais non limitativement, l'invention sera utilisée auprès de patientes au niveau vaginal, périnéal et anal en prévention de la douleur induite par des sutures, des cicatrices et des plaies, ainsi que toute autre douleur, notamment induite par la luxation ou une fracture du coccyx.

Selon des modes de réalisation alternatifs, l'invention sera utilisée auprès de patients essentiellement, mais non limitativement, au niveau anal dans un cadre similaire de prévention.

L'invention concerne particulièrement un dispositif de coque protectrice ergonomique de type orthèse, destinée à être placée au niveau de l'entrejambe d'un patient, de préférence d'une patiente.

De nombreuses circonstances peuvent provoquer des douleurs à l'entrejambe d'un patient ou d'une patiente. A titre d'exemple, suite à un accouchement, une femme peut avoir reçu des points de suture après une déchirure des tissus ou une épisiotomie, mais avoir des éraillures vulvaires ou anales. Dans d'autres cas, une patiente peut souffrir d'hémorroïdes, d'hématomes ainsi qu'en raison d'une luxation du coccyx. De telles douleurs peuvent aussi provenir de traitements de condylomes périnéaux et vulvaires, ou bien peuvent être provoquées suite à une intervention sur le périnée suite à une bartholinite, une plastie vulvaire ou des cures de prolapsus par voie basse.

Si ces douleurs sont supportables en statures débout ou allongée, elles se manifestent de façon contraignante et difficilement supportable en position assise, appliquant une pression sur les zones sensibles.

Dans certains cas, ces pressions sont néfastes pour le bon rétablissement du patient ou de la patiente, notamment la cicatrisation éventuelle des tissus, et peuvent aller jusqu'à provoquer des complications ou bien des pathologies connexes, comme des douleurs articulaires au niveau du dos et des épaules, en raison d'un mauvais positionnement assis. Suite à un accouchement et lors de l'allaitement, cette malposition peut aussi provoquer des crevasses mamelonnaires, une mauvaise vidange des seins, des engorgements douloureux ou bien des mastites.

De nombreux dispositifs de coque protectrice ergonomique de type orthèse périnéale permettent de diminuer les douleurs à l'entrejambe et sont décrits pour de diverses applications comme par exemple : des dispositifs de protection des parties génitales au cours d'une activité physique US 4,186,739, US 5,483,705 et US 2006/0229546 ; des selles de vélo ergonomiques protégeant la zone périnéale du cycliste US 5,978,970 (le préambule de la revendication 1 annexée se fondant sur ce document) et US 2008/0229486 ; des assises permettant de réduire les troubles du dos en diminuant la pression au niveau de la zone périnéale US 2002/0124318 et EP 2 191 746 ; et des dispositifs médicaux permettant de protéger la zone périnéale CN 201127648 et EP 0 448 336.

De plus, il existe une autre solution précaire sous la forme d'un coussin gonflable de forme annulaire, ménageant un espace libre central et offrant un appui périphérique sur lequel le patient ou la patiente s'assoie. Un tel coussin est généralement gonflé pour le remplir d'air. Mais il n'apporte toutefois pas entière satisfaction, en raison de son peu d'ergonomie, entraînant son déplacement de la zone à protéger par glissement. De plus, un tel coussin perd régulièrement de sa pression et doit être regonflé périodiquement. Enfin, il est volumineux et encombrant, très peu pratique une fois en place.

La présente invention propose une alternative qui a pour but de pallier les inconvénients de l'état de la technique, en proposant un dispositif de coque protectrice ergonomique de type orthèse destinée à être placée au niveau d'une entrejambe d'un patient ou d'une patiente.

Une telle coque présente une forme à même de la positionner et la maintenir au niveau de l'entrejambe, tout en autorisant des appuis sur des zones anatomiques distantes des zones sensibles objets de la douleur potentielle.
De plus, son caractère ergonomique est particulièrement adapté à la morphologie du bassin, masculin ou féminin, ses dimensions pouvant être adaptées en fonction de la corpulence , de la taille et du sexe de la personne.

Pour ce faire, un tel dispositif, défini dans la revendication 1, est constitué d'une structure pourvue d'une extrémité antérieure destinée à être positionnée vers l'avant de l'entrejambe et d'une extrémité postérieure destinée à être positionnée vers l'arrière de l'entrejambe, cette structure présentant une forme symétrique selon un plan médian vertical longitudinal et présentant une forme évasée croissante depuis l'extrémité antérieure vers l'extrémité postérieure. De plus la structure comprend au moins une cavité s'étendant de l'une à l'autre des extrémités antérieure et postérieure. Le dispositif se caractérise par le fait que la cavité s'étend de l'une à l'autre des extrémités en suivant la forme évasée, et comprend un rétrécissement au niveau de l'extrémité postérieure évasée, ladite structure comprenant des zones rigides d'appui ménagées au niveau de l'extrémité postérieure évasée de part et d'autre du rétrécissement.

Ainsi, un patient portant ou une patiente équipée de l'orthèse selon l'invention peut s'assoir plus confortablement, mais aussi le passage vers la position assise est facilité et rendu moins douloureux.

De plus, selon d'autres caractéristiques non limitatives les zones d'appui peuvent être constituées par une épaisseur pleine depuis le fond de la cavité.

Selon un mode de réalisation préférentielle, la cavité peut présenter un fond arrondi.

De préférence, la cavité peut comprendre en face inférieure un maillage de renforcement selon des arêtes saillantes extérieurement et sécantes entre elles.

En particulier, la cavité peut comprendre des orifices traversant son fond répartis sur sa longueur.

En outre, la forme globalement concave de cette orthèse forme un bac de récupération d'éventuels fluides corporels et autres sécrétions, comme des lochies, permettant leur
canalisation et leur évacuation, notamment par l'intermédiaire d'orifices, ces derniers pouvant être situés en vis-à-vis du vagin dans la version féminine d'une telle orthèse.

Selon un mode de réalisation, la structure peut comprendre au moins une frange périphérique de contact s'étendant horizontalement en saillie au moins sur une partie du pourtour en face supérieure de ladite structure.

Selon un autre mode de réalisation, ladite structure peut être constituée d'un unique élément réalisé en un matériau siliconé semi-rigide.

Selon encore un autre mode de réalisation, prévu pour l'homme, que l'extrémité antérieure peut comprendre une ouverture.

De préférence, dans cette configuration masculine, la structure peut comprendre au niveau de l'extrémité antérieure des moyens de fixation se présentant sous la forme d'au moins une paire de brins s'étendant en vis-à-vis de part et d'autre de la cavité, les brins présentant à leur extrémité libre (103) des moyens de connexion.

Dès lors, la forme combinée à une structure en matériau spécifique permet d'offrir une zone d'appui plus rigide que le reste de l'orthèse, améliorant son application au contact du corps de la patiente et épousant parfaitement sa morphologie.

D'autres caractéristiques et avantages de l'invention ressortiront de la description détaillée qui va suivre des modes de réalisation non limitatifs de l'invention, en référence aux figures annexées, dans lesquelles :
- la figure 1 représente en perspective depuis une vue arrière de dessus d'un mode spécifique de réalisation d'un dispositif d'orthèse selon l'invention, une telle orthèse étant destinée à une patiente ;
- la figure 2 représente en perspective une vue avant de dessus de la figure 1 ;
- la figure 3 représente une vue plane de dessus d'un mode spécifique de réalisation ;
- la figure 4 représente une vue plane selon une coupe verticale longitudinale médiane de la figure 3 ;
- la figure 5 représente une vue plane selon une coupe verticale transversale de la figure 3 selon un axe A-A' ;
- la figure 6 représente une vue plane selon une coupe verticale transversale de la figure 3 selon un axe B-B' ;
- la figure 7 représente en perspective une vue de dessous du mode préférentiel de réalisation ;
- la figure 8 représente en perspective une vue de dessus de la figure 7 ;
- la figure 9 représente en perspective de trois-quarts avant une vue de dessus d'un autre mode de réalisation d'orthèse destinée à un patient ; et
- la figure 10 représente un vue en plan de dessous de la figure 9.

La présente invention concerne un dispositif 1 de coque protectrice ergonomique de type orthèse.

Un tel dispositif 1 est constitué d'une structure prévue au moins partiellement souple et creuse, de manière à venir être accolée contre la peau d'un patient ou d'une patiente au niveau de son entrejambe, se déformant pour épouser son anatomie, sans pour autant venir au contact et en appui de zones douloureuses induites par les raisons précédemment évoquées. En particulier, la forme de la structure présente un espace central creux, qui, une fois l'orthèse mise en place, se retrouve en vis-à-vis de la zone anatomique visée s'étendant pour l'homme depuis l'arrière des testicules jusque sous l'anus et pour la femme depuis l'ouverture vaginale jusqu'à l'anus et empêche le contact et l'appui à ce niveau.

Pour ce faire, tout d'abord, ladite structure est pourvue d'une extrémité antérieure 2, destinées à être positionnée vers l'avant pour l'homme au derrière des testicules et pour la femme au niveau du vagin ou de l'abdomen de la patiente, et d'une extrémité postérieure 3, destinée à être positionnée vers l'arrière au niveau de l'anus ou du fessier.

En outre, ladite structure présente une face supérieure 4 et une face inférieure 5, ladite face supérieure 4 étant destinée à venir être accolée contre la peau lors de sa mise en place.

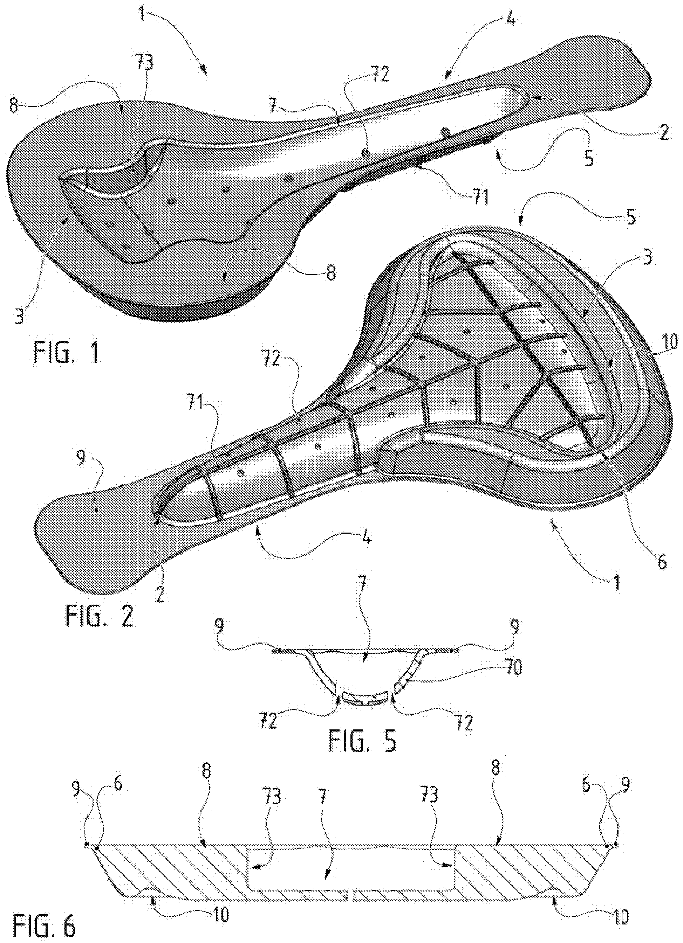

Tout d'abord, pour la femme, comme représenté sur les figures 1 à 9, cette structure présente une forme générale oblongue et allongée. Plus précisément, cette forme est évasée et croissante depuis l'extrémité antérieure 2 vers l'extrémité postérieure 3. En somme, les parois longitudinales s'étendant latéralement vont en s'écartant depuis l'avant vers l'arrière, la structure étant plus large à ce dernier endroit.

On notera que cet évasement peut être rectiligne ou sensiblement rectiligne le long d'une portion avant de la structure et courbe ou incurvé, avec une inflexion ou une croissance plus importante au niveau de l'autre portion située à l'arrière. De plus, au niveau de cette dernière et de l'extrémité postérieure, le bord arrière de la structure peut aussi être arrondi selon un arc de cercle reliant deux extrémités latérales situées au plus large de l'évasement.

Par ailleurs, cette forme est symétrique selon un plan médian vertical longitudinal.

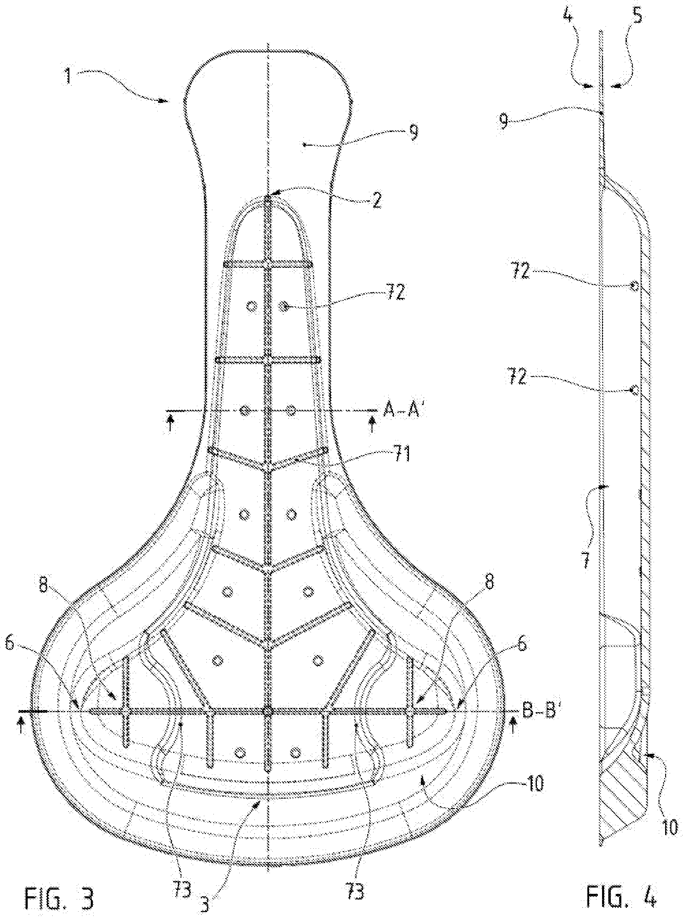

Un exemple de réalisation d'une telle forme est visible sur la figure 4.

Ainsi, ladite structure présente alors sensiblement et globalement une forme de selle de cycle, ou d'un T arrondi et inversé, coïncidant avec la morphologie humaine au niveau de l'entrejambe et permettant d'être positionné à cet endroit.

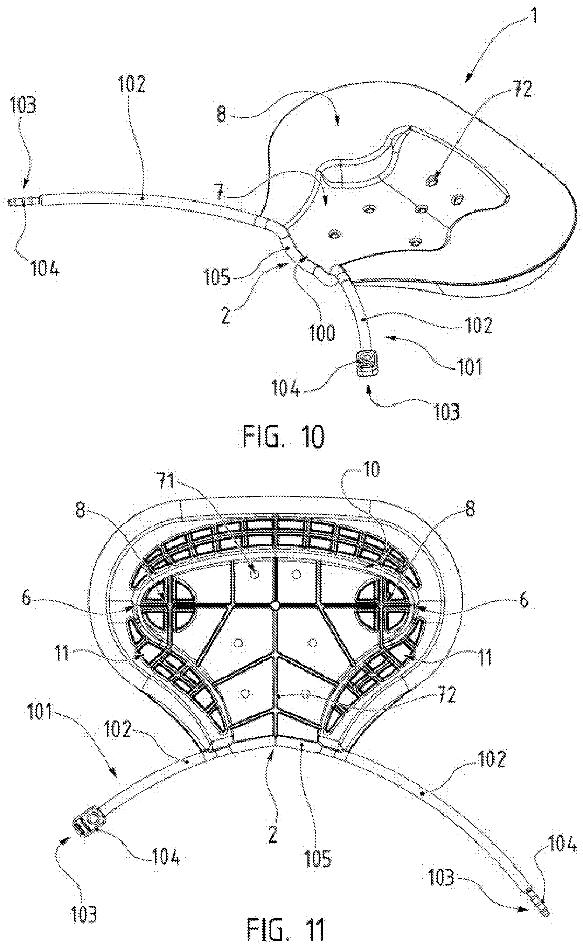

Par ailleurs, pour l'homme, comme représenté sur les figures 10 et 11, la structure du dispositif 1 d'orthèse est raccourcie, ne présentant pas une forme allongée, mais plus compacte. En d'autres termes, l'extrémité antérieure 2 ne se retrouve pas déportée vers l'avant, mais reculée en arrière, pour venir coïncider avec l'anatomie génitale masculine.

Dès lors, la forme évasée de la structure de l'orthèse pour homme croit plus fortement depuis cette extrémité antérieure 2 vers l'extrémité postérieure 3. En somme, les parois longitudinales s'étendant latéralement vont en s'écartant depuis l'avant vers l'arrière selon un angle initial plus important, notamment supérieur à 45°, par rapport à l'axe médian longitudinal.

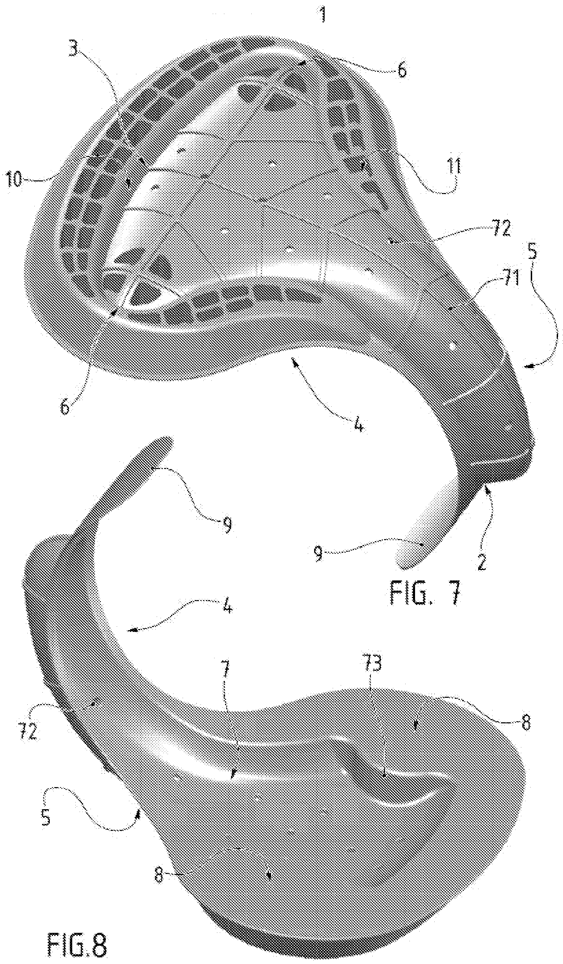

Selon les modes de réalisation représentés sur les figures 1 à 6 ainsi que 10 et 11, la structure est présentée à plat. Selon un autre mode de réalisation, représenté sur les figures 7 et 8, ladite structure peut présenter une forme arquée ou incurvée, de forme alors concave au niveau de sa face supérieure 4, de manière à épouser le galbe arrondi de l'anatomie.

Selon une caractéristique essentielle, ladite structure est prévue creuse, afin de ne pas être en contact avec des zones anatomiques douloureuses.

Pour ce faire, ladite structure comprend au moins une cavité 7 s'étendant de l'une à l'autre desdites extrémités antérieure 2 et postérieure 3 en suivant ladite forme évasée. En somme, la structure présente, vue depuis le dessus, des parois concaves ménageant au moins un espace creux.

En particulier, selon le mode préférentiel de réalisation, particulièrement visible sur les figures 1, 2 et 5, la structure présente au niveau de la cavité 7 une unique paroi arrondie 70. En somme, ladite cavité 7 présente un fond arrondi. En outre, ce fond a une section d'arc de cercle, de corde plus ou moins longue en fonction de l'endroit où il est situé par rapport à l'évasement, réciproquement à l'arrière ou à l'avant.

De plus, dans le mode de réalisation masculin, ladite extrémité antérieure 2 peut présenter une ouverture 100. Le chant périphérique de cette dernière va alors venir au contact de la face postérieure du scrotum, assurant ainsi la fermeture de la cavité 7.

Selon un mode de réalisation masculine alternatif, non représenté, l'ouverture 100 de l'extrémité antérieure 2 peut être obturée par une paroi. Cette dernière peut être prévu souple et incurvée, présentant une forme ergonomique pour venir au contact des testicules, au niveau de la face postérieure du scrotum.

De plus, dans le mode masculin de réalisation, la structure du dispositif 1 comprend des moyens 101 de fixation destinés à assujettir l'orthèse et la rendre solidaire de l'anatomie de l'homme. De préférence, ces moyens 101 permettent de fixer le dispositif 1 au niveau de la base de la verge.

Selon le mode préférentiel de réalisation, les moyens de fixation 101 se situent au niveau de l'extrémité antérieure 2. En particulier, ils peuvent être constitués par au moins une parie de brins 102 s'étendant en vis-à-vis, de part et d'autre de la cavité 7, au niveau de ladite ouverture 100. De tels brins 102 peuvent avoir une section ronde ou aplatie, formant dans ce dernier cas des sangles. Ces brins 102 présentent à leur extrémité libre 103 des moyens de connexion 104. Ces derniers permettent de fixer ensemble lesdites extrémités 103, formant alors une boucle fermée.

De plus, les moyens de connexion 104 peuvent être prévus réglable, afin d'ajuster le diamètre de cette boucle, en particulier d'augmenter ou de rétrécir la taille de ladite boucle, en fonction de l'anatomie de la verge du patient.

Selon le mode préférentiel de réalisation, représenté sur les figures 10 et 11, lesdits moyens de connexion 104 peuvent être constitués, à l'extrémité 103 d'un premier brin 102 par une section de moindre diamètre, formant une partie mâle, destinée à coopérer en emboîtement au sein d'un orifice, formant une partie femelle de forme et dimensions complémentaires, dont est pourvu l'extrémité 103 d'un second brin 102.

En outre, ladite section de moindre diamètre peut être pourvue de rendent, notamment de forme annulaire, répartie régulièrement sur la longueur de l'extrémité 103 dudit premier brin 102. Ces anneaux permettent d'indexer des positions en les introduisant en force au sein dudit orifice de la partie femelle du second brin opposé 102, formant ainsi des butées.

On notera que les brins 102 peuvent former un bourrelet 105 au niveau et le long du chant de l'ouverture 100, améliorant le contact contre le scrotum.

En outre, lesdits brins 102 peuvent être préférentiellement prévus souples. Ils peuvent être constitués du même matériau que le reste du dispositif 1.

Selon une caractéristique additionnelle, pour tout dispositif 1 d'orthèse pour homme ou pour femme, comme visible sur la figure 2, ladite cavité 7 peut comprendre en face inférieure un maillage de renforcement selon des arêtes 71 saillantes extérieurement et sécantes entre elles. En particulier, une arête centrale peut s'étendre selon l'axe médian de symétrie, tandis que d'autres arêtes viennent la rejoindre en croissant leur direction respective. Une autre arête transversale peut s'étendre en passant par les extrémités latérales 6.

En outre, ce maillage permet de rigidifier la structure du dispositif 1, tout en autorisant sa déformation.

A ce titre, ladite structure est préférentiellement constituée d'un unique élément réalisé en un matériau siliconé semi-rigide. En somme, ce matériau assure une souplesse, une élasticité et une mémoire de forme à l'orthèse, autorisant sa déformation pour venir être apposée et épouser l'anatomie, puis revenir en position d'origine, comme visible sur les figures 1 et 2, notamment en vue de son nettoyage et de son stockage.

De préférence, ledit matériau peut être un silicone biocompatible et microporeux. De préférence, ledit silicone peut être un silicone médical antibactérien.

Dès lors, ledit maillage assure le maintien en forme de la structure au regard de la souplesse du matériau la constituant, en particulier dans le cas du silicone qui est un matériau très souple. Ainsi, même déformée et positionnée au niveau de l'entrejambe, la structure conserve le creux de la cavité 7.

A ce titre, le creux permet de récupérer des écoulements éventuels de fluides corporels, notamment des lochies. Afin d'éviter que ces fluides stagnes au sein de la cavité 7, la cavité 7 prévoit de canaliser lesdits fluides vers l'extérieur. Pour ce faire, d'une part, la forme arrondie de la cavité 7 permet de guider l'écoulement vers sa partie basse. D'autre part, ladite cavité 7 peut comprendre des orifices 72 traversant son fond répartis sur sa longueur, de préférence de part et d'autre du plan médian de symétrie.

Avantageusement, le dispositif 1 prévoit d'offrir des zones d'appui destinées à être positionnées en vis-à-vis de deux zones anatomiques situées de part et d'autre du périnée entre l'orifice anal et l'ouverture vaginale pour la femme ou entre l'arrière du scrotum pour l'homme, au niveau du périnée postérieur et à proximité des tubérosités ischiatiques.

Pour ce faire, ladite cavité comprend un rétrécissement au niveau de l'extrémité postérieure 3 évasée. En somme, à l'endroit arrière le plus large de la structure, la largeur de la cavité 7 est présente une diminution, comme visible sur la figure 6.

En particulier, cette diminution peut être obtenue par l'intermédiaire de renfoncements 73. Ces derniers peuvent présenter une forme arrondie, convexe, comme visible sur la figure 3.

Ce rétrécissement permet alors de ménager de part et d'autre de la cavité 7 deux parties plus épaisses destinées à recevoir en appui les zones anatomiques susmentionnées, sans occasionner de pression au niveau des zones sensibles et douloureuses du périnée et de l'anus.

Ainsi, ladite structure comprend des zones rigides d'appui 8 ménagées au niveau de ladite extrémité postérieure 3 évasée de part et d'autre dudit rétrécissement.

De plus, lesdites zones d'appui 8 peuvent être constituées par une épaisseur pleine depuis le fond de ladite cavité 7. En somme, sous les zones 8, la cavité 7 est remplie. Ce remplissage peut être réalisé avec le même matériau que celui constituant l'intégralité du dispositif 1, mais aussi part un matériau différent, par exemple un gel, offrant résistance mais aussi souplesse. Dans ce dernier cas, le matériau différent est enfermé dans une poche ménagée entre les parois latérales de la cavité 7 au niveau de chaque renfoncement 73, la paroi supérieure et la paroi inférieure de la structure.

Selon un autre mode de réalisation, lesdites zones d'appui 8 peuvent être prévue partiellement creuses, notamment alvéolaires. Ces alvéoles peuvent notamment être sous forme d'un nid d'abeille. En somme, l'intérieur de ces zones 8 est creux et entretoisé de parois assurant la rigidité désirée. Cette aération intérieure permet aussi de diminuer d'au moins 20 à 25 % le poids du dispositif 1.

Selon le mode de réalisation représenté sur la figure 8, l'épaisseur du reste de la structure au niveau de sa partie postérieure est aussi prévue alvéolaire, lesdites alvéoles 11 formant un treillis de renfort, limitant la déformation du dispositif 1 lorsqu'il est en place, notamment en appui lorsque le patient se met en position assise. Lesdites alvéoles 11 peuvent être réparties autour des zones d'appui 8 et de la concavité 7.

On notera que ces alvéoles 11 peuvent être prévues de manière à déboucher en face inférieure 5 dudit dispositif 1.

Selon une caractéristique additionnelle, afin d'améliorer la structure du dispositif 1 et de lui conférer la flexibilité et le maintien en flexibilité, sans se déformer outre mesure dans des configurations non désirées, ladite structure peut comprendre au niveau de sa partie arrière au moins une gorge 10. Cette dernière peut s'étendre au moins en partie autour de la partie arrière de la structure, de part et d'autre des zones d'appui 8. De plus, ladite gorge 10 est ménagée en face inférieure 5, sous forme d'au moins un évidement conformé globalement en V inversé. Ainsi, le fond de cette gorge 10 forme une arête intérieurement à ladite structure, augmentant sa rigidité.

Selon le mode de réalisation représenté sur la figure 2, la gorge 10 comprend deux extrémités situées au niveau de l'évasement de la partie arrière de la structure. A partir de ces extrémités, la gorge 10 s'écarte vers l'arrière, à savoir que la largeur de son ouverture va croissante, puis se rétrécit jusqu'à de nouveau se joindre aux niveaux de chaque extrémité latérale 6. De plus, une portion de la gorge 10 se poursuit au niveau de l'extrémité arrière 3 de la structure, formant un arc orienté sensiblement orthogonalement par rapport à l'axe médian longitudinal du dispositif 1. Enfin, cette portion de gorge va s'écartant depuis les extrémités 6 jusqu'au centre au niveau dudit axe.

Selon une caractéristique additionnelle, le mode de réalisation préférentiel, représenté sur les figures, prévoit que la structure peut comprendre au moins une frange 9 périphérique de contact s'étendant horizontalement en saillie au moins sur une partie du pourtour en face supérieure de ladite structure. Une telle frange assure, d'une part, un contact avec la peau et le maintien de la structure, notamment par adjonction d'adhésif directement sur la peau, et, d'autre part, l'étanchéité du dispositif 1, évitant les écoulements de sortir de la cavité 7.

En outre, cette frange peut avoir une largeur différente à différent niveau de la périphérie de la structure, plus allongée vers l'avant, sensiblement équivalente sur tout le pourtour de la partie arrière évasée, mais moins large, jusqu'à inexistante au niveau de la partie médiane ou centrale. En effet, cette dernière est destinée à venir se loger au niveau de l'intérieur des cuisses où l'espace est le plus restreint.

Ainsi, le dispositif 1 selon l'invention permet, du fait de la forme et la constitution de sa structure, un appui localisé à distance des zones anatomiques sensibles et douloureuses, tout en évitant le contact avec ces dernières disposées en vis-à-vis de la cavité 7, offrant une orthèse améliorant le confort et protégeant lesdites zones de tout pression.

## Revendications

1. Dispositif (1) de coque protectrice ergonomique de type orthèse qui est destiné à être placé au niveau d'une entrejambe d'un patient ou d'une patiente, le dispositif (1) étant constitué d'une structure pourvue d'une extrémité antérieure (2) destinée à être positionnée vers l'avant de l'entrejambe et d'une extrémité postérieure (3) destinée à être positionnée vers l'arrière de l'entrejambe au niveau de l'anus ou du fessier, cette structure présentant une forme symétrique selon un plan médian vertical longitudinal et présentant une forme évasée croissante depuis l'extrémité antérieure (2) vers l'extrémité postérieure (3), ls structure comprenant au moins une cavité (7) s'étendant de l'une à l'autre des extrémités antérieure (2) et postérieure (3)
**caractérisé par le fait que** la cavité (7) s'étend de l'une à l'autre des extrémités en suivant la forme évasée, et comprend un rétrécissement au niveau de l'extrémité postérieure (3) évasée, ladite structure comprenant des zones (8) rigides d'appui ménagées au niveau de l'extrémité postérieure (3) évasée de part et d'autre du rétrécissement.

2. Dispositif de coque (1) selon la revendication 1, **caractérisé par le fait que** les zones d'appui (8) sont constituées par une épaisseur pleine depuis le fond de la cavité (7).

3. Dispositif de coque (1) selon l'une quelconques des revendications précédentes, **caractérisé par le fait que** la cavité (7) présente un fond arrondi.

4. Dispositif de coque (1) selon l'une quelconque des revendications précédentes, **caractérisé par le fait que** la cavité (7) comprend en face inférieure un maillage de renforcement selon des arêtes (71) saillantes extérieurement et sécantes entre elles.

5. Dispositif de coque (1) selon l'une quelconque des revendications précédentes, **caractérisé par le fait que** la cavité (7) comprend des orifices (72) traversant son fond répartis sur sa longueur.

6. Dispositif de coque (1) selon l'une quelconque des revendications précédentes, **caractérisé par le fait que** la structure comprend au moins une frange périphérique (9) de contact s'étendant horizontalement en saillie au moins sur une partie du pourtour en face supérieure de ladite structure.

7. Dispositif de coque (1) selon l'une quelconque des revendications précédentes, **caractérisé par le fait que** ladite structure est constituée d'un unique élément réalisé en un matériau siliconé semi-rigide.

8. Dispositif de coque (1) selon l'une quelconque des revendications précédentes, **caractérisé par le fait que** ladite structure présente une forme arquée ou incurvée en face supérieure (4).

9. Dispositif de coque (1) selon l'une quelconque des revendications précédentes, **caractérisé par le fait que** l'extrémité antérieure (2) comprend une ouverture (100)

10. Dispositif de coque (1) selon la revendication 9, **caractérisé par le fait que** la structure comprend au niveau de l'extrémité antérieure (2) des moyens de fixation (101) se présentant sous la forme d'au moins une paire de brins (102) s'étendant en vis-à-vis de part et d'autre de la cavité (7), les brins (102) comportant à leur extrémité libre (103) des moyens de connexion (104).

## Patentansprüche

1. Ergonomische Schutzschalenvorrichtung (1) der Art Orthese, die dazu bestimmt ist, im Bereich eines Schritts eines Patienten angebracht zu werden, wobei die Vorrichtung (1) aus einer Struktur besteht, die mit einem vorderen Ende (2), das dazu bestimmt ist, nach vorne am Schritt positioniert zu werden, und einem hinteren Ende (3), das dazu bestimmt ist, nach hinten am Schritt, im Bereich des Anus oder des Gesäßes positioniert zu werden, versehen ist, wobei diese Struktur eine symmetrische Form entlang einer vertikalen Längsmittelebene aufweist und eine von dem vorderen Ende (2) zum hinteren Ende (3) zunehmend aufgeweitete Form aufweist, wobei die Struktur mindestens einen Hohlraum (7) aufweist, der sich von dem einen zum anderen der vorderen (2) und hinteren (3) Enden erstreckt, **dadurch gekennzeichnet, dass** sich der Hohlraum (7) entlang der aufgemeiteten Form vom einen zum anderen Ende erstreckt, und eine Verengung im Bereich des aufgeweiteten hinteren Endes (3) umfasst, wobei die besagte Struktur starre Abstützzonen (8) umfasst, die im Bereich des aufgeweiteten hinteren Endes (3) auf beiden Seiten der Verengung vorgesehen sind.

2. Schalenvorrichtung (1) nach Anspruch 1, **dadurch gekennzeichnet, daß** die Abstützzonen (8) aus einer vollwändigen Dicke ab dem Boden des Hohlraums (7) bestehen.

3. Schalenvorrichtung (1) nach irgendeinem der vorhergehenden Ansprüche, **dadurch gekennzeichnet, dass** der Hohlraum (7) einen abgerundeten Boden aufweist.

4. Schalenvorrichtung (1) nach irgendeinem der vorhergehenden Ansprüche, **dadurch gekennzeichnet, dass** der Hohlraum (7) auf der unteren Fläche einen Verstärkungs-Maschendraht entlang nach außen vorstehenden und sich gegenseitig schneidenden Kanten (71) umfasst.

5. Schalenvorrichtung (1) nach irgendeinem der vorhergehenden Ansprüche, **dadurch gekennzeichnet, dass** der Hohlraum (7) Öffnungen (72) umfassst, die durch seinen Boden hindurch gehen und über seine Länge verteilt sind.

6. Schalenvorrichtung (1) nach irgendeinem der vorhergehenden Ansprüche, **dadurch gekennzeichnet, dass** die Struktur mindestens einen Umfangskontaktstreifen (9) umfasst, der sich horizontal an der oberen Fläche der besagten Struktur zumindest auf einem Teil des Umfangs vorspringend erstreckt.

7. Schalenvorrichtung (1) nach irgendeinem der vorhergehenden Ansprüche, **dadurch gekennzeichnet, dass** die besagte Struktur aus einem einzigen Element aus einem halbsteifen Silikonmaterial besteht.

8. Schalenvorrichtung (1) nach irgendeinem der vorhergehenden Ansprüche, **dadurch gekennzeichnet, dass** die besagte Struktur an der oberen Fläche (4) eine bogenförmige oder gekrümmte Form aufweist.

9. Schalenvorrichtung (1) nach irgendeinem der vorhergehenden Ansprüche, **dadurch gekennzeichnet, dass** das vordere Ende (2) eine Öffnung (100) umfasst.

10. Schalenvorrichtung (1) nach Anspruch 9, **dadurch gekennzeichnet, dass** die Struktur im Bereich des vorderen Endes (2) Befestigungsmittel (101) umfasst, die als mindestens ein Paar von sich gegenüberliegend und auf beiden Seiten des Hohlraums (7) erstreckenden Strängen (102) ausgestaltet sind, wobei die Stränge (102) an ihrem freien Ende (103) verbindungsmittel (104) umfassen.

## Claims

1. An ergonomic protective shell device (1) such as an orthosis, which is intended to be placed at the level of a crotch of a patient, the device (1) being comprised of a structure provided with a front end (2) intended to be positioned towards the front of the crotch, and a rear end (3) intended to be positioned towards the rear of the crotch, at the level of the anus or the buttocks, this structure having a symmetrical shape in a longitudinal vertical median plane and having a flared shape increasing from the front end (2) to the rear end (3), the structure comprising at least one cavity (7) extending from one to the other of the front (2) and rear (3) ends, wherein the cavity (7) extends from one to the other end according to the flared shape, and comprises a narrowing at the level of the flared rear end (3), said structure comprising rigid support areas (8) provided for at the level of the flared rear end (3) on both sides of the narrowing.

2. The shell device (1) according to claim 1, wherein the support areas (8) are formed of a massive thickness from the bottom of the cavity (7).

3. The shell device (1) according to any one of the preceding claims, wherein the cavity (7) has a rounded bottom.

4. The shell device (1) according to any one of the preceding claims, wherein the cavity (7) comprises on the lower face a reinforcing mesh according to edges (71) projecting externally and intersecting with each other.

5. The shell device (1) according to any one of the preceding claims, wherein the cavity (7) comprises orifices (72) passing through its bottom, distributed over its length.

6. The shell device (1) according to any one of the preceding claims, wherein the structure comprises at least one peripheral contact fringe (9) extending horizontally and projecting at least over a part of the periphery on the front face of said structure.

7. The shell device (1) according to any one of the preceding claims, wherein said structure is formed of a single element made out of a semi-rigid silicone material.

8. The shell device (1) according to any one of the preceding claims, wherein said structure has an arcuate or curved shape on the upper face (4).

9. The shell device (1) according to any one of the preceding claims, wherein the front end (2) comprises an opening (100).

10. The shell device (1) according to claim 9, wherein the structure comprises, at the level of the front end (2), fastening means (101) being in the form of at least one pair of strands (102) extending in front of each other on both sides of the cavity (7), the strands (102) including connection means (104) at their free end (103).
